(19) **Europäisches Patentamt European Patent Office Office européen des brevets**

(11) **EP 3 424 451 B1**

(12) **EUROPEAN PATENT SPECIFICATION**

(45) Date of publication and mention of the grant of the patent:
**20.07.2022 Bulletin 2022/29**

(21) Application number: **17760007.9**

(22) Date of filing: **28.02.2017**

(51) International Patent Classification (IPC):
**A61B 17/80** (2006.01)    **A61L 27/04** (2006.01)
**A61L 27/42** (2006.01)    **A61L 27/06** (2006.01)
**A61L 27/30** (2006.01)    **A61L 27/50** (2006.01)
**A61L 27/54** (2006.01)    **A61L 27/56** (2006.01)
**A61L 31/02** (2006.01)    **A61L 31/08** (2006.01)
**A61L 31/14** (2006.01)    **A61L 31/16** (2006.01)

(52) Cooperative Patent Classification (CPC):
**A61B 17/80; A61L 27/06; A61L 27/306;
A61L 27/50; A61L 27/54; A61L 27/56;
A61L 31/028; A61L 31/088; A61L 31/14;
A61L 31/146; A61L 31/16;** A61L 2300/102;
A61L 2300/404; A61L 2300/602

(86) International application number:
**PCT/JP2017/007822**

(87) International publication number:
**WO 2017/150542 (08.09.2017 Gazette 2017/36)**

(54) **ANTIBACTERIAL DEVICE TO BE IMPLANTED IN VIVO**

ANTIBAKTERIELLE VORRICHTUNG ZUR IN-VIVO-IMPLANTATION

INSTRUMENT ANTIBACTÉRIEN DESTINÉ À ÊTRE IMPLANTÉ DANS UN ORGANISME VIVANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.02.2016 JP 2016038141**

(43) Date of publication of application:
**09.01.2019 Bulletin 2019/02**

(73) Proprietor: **Medtronic Sofamor Danek, Co., Ltd. Osaka-shi, Osaka 553-0003 (JP)**

(72) Inventors:
• **SUGINO, Atsushi**
  **Osaka-shi**
  **Osaka 553-0003 (JP)**
• **FUKUZAKI, Satoshi**
  **Tsu-shi**
  **Mie 514-8507 (JP)**

(74) Representative: **FRKelly
27 Clyde Road
Dublin D04 F838 (IE)**

(56) References cited:
WO-A1-2015/148800    JP-A- 2002 536 048
JP-A- 2013 528 411    JP-B2- 5 175 185
US-A- 4 476 590    US-A1- 2012 123 485
US-A1- 2015 182 645    US-B1- 8 927 004

• **KEUN-TAEK OH ET AL: "Electrochemical properties of suprastructures galvanically coupled to a titanium implant", JOURNAL OF BIOMEDICAL MATERIALS RESEARCH PART B: APPLIED BIOMATERIALS, vol. 70B, no. 2, 6 April 2004 (2004-04-06) , pages 1-27, XP055624741,**
• **FERRARIS S ET AL: "Antibacterial titanium surfaces for medical implants", MATERIALS SCIENCE AND ENGINEERING C, ELSEVIER SCIENCE S.A, CH, vol. 61, 31 December 2015 (2015-12-31), pages 965-978, XP029403060, ISSN: 0928-4931, DOI: 10.1016/J.MSEC.2015.12.062**
• **F. Mansfeld ET AL: "Galvanic corrosion of Al alloys-III. The effect of area ratio", CORROSION SCIENCE., vol. 15, no. 4, 1 January 1975 (1975-01-01), pages 239-250, XP055737717, GB ISSN: 0010-938X, DOI: 10.1016/S0010-938X(75)80019-9**

**Description**

TECHNICAL FIELD

The present invention relates to an implant device, and specifically relates to an implant device that achieves an antimicrobial property while it is implanted in vivo for use.

BACKGROUND ART

A variety of instruments such as cages, wires, plates, screws, rods, connectors, crosslinks, hooks, set screws, artificial vertebral bodies, and artificial intervertebral discs are used as orthopedic implant devices. These instruments may be contaminated with microorganisms when they are implanted in vivo for use. If an implant device is colonized by microorganisms, it is very difficult to prevent microbial growth. To prevent microbial growth and infection in the body, it is necessary to perform surgery to remove the implant device for replacement with a new one, which places an enormous burden on the patient. It is desired that an antimicrobial implant device be provided.

As a technique for imparting an antimicrobial property to an implant device, a medical implant system has been proposed (Patent Literature 1) which comprises a metal component containing an antimicrobial metal disposed on an external surface of the implant body; a power source having a first terminal and a second terminal, one of the terminals being in electrical communication with the metal component; and an insulator placed in a current path between the first terminal of the power source and the second terminal of the power source, preventing current flowing from the first terminal from reaching the second terminal without completing a circuit including a conductive body tissue or fluid adjacent to the external surface of the implant system when implanted. Patent Literature 1 discloses that examples of the antimicrobial metal include silver, copper, both silver and copper, both silver and cadmium, and a combination of silver, copper, and cadmium. In Patent Literature 1, the external power source equipment and the terminals are required in addition to the implant body to achieve an antimicrobial property, and a treatment or an external operation is needed to impart an antimicrobial property. US 4 476 590 A discloses an orthopedic plate comprising a titanium or titanium alloy porous substrate coated with silver. The silver is released and has antimicrobial activity. Combination with more noble metals is disclosed to improve galvanic release. Further improvement is required from the viewpoint of reducing the burden on patients.

CITATION LIST

PATENT LITERATURE

PTL 1: Japanese Patent No. 5175185

SUMMARY OF INVENTION

TECHNICAL PROBLEM

It is an object of the present invention to provide an implant device that can achieve an antimicrobial property, simply by being implanted in vivo, to reduce the burden on patients.

SOLUTION TO PROBLEM

The present invention is directed to an antimicrobial implant device as defined in claim 1.

ADVANTAGEOUS EFFECTS OF INVENTION

The implant device of the present invention, once implanted in vivo, can achieve an antimicrobial property without requiring an external operation or treatment, thereby significantly reducing the burden on patients.

BRIEF DESCRIPTION OF DRAWINGS

Figs. 1(a) and (b) are a plan view and a cross-sectional view, respectively, of a plate 1.
Figs. 2(a) and (b) are a plan view and a cross-sectional view, respectively, of the plate 1 of another embodiment.

Figs. 3(a) and (b) are a plan view and a cross-sectional view, respectively, of the plate 1 of still another embodiment.
Figs. 4(a) and (b) are a plan view and a cross-sectional view, respectively, of the plate 1 of yet another embodiment.

DESCRIPTION OF EMBODIMENTS

The present invention will be described with reference to the attached drawings.

An antimicrobial implant device of the present invention is an implant device made of a first material and a second material, wherein at least a surface of the first material comprises an antimicrobial metal; at least a surface of the second material is nobler than the first material; an electric circuit is formed between the first material and the second material when the device is implanted in vivo for use; and ions of the antimicrobial metal are eluted from the first material under a presence of an electrolyte to impart an antimicrobial property to a surface and/or a surrounding area of the first material. The antimicrobial implant device of the present invention is made of at least two materials, and simply because these two materials are contacted in vivo, a short circuit occurs to form an electric circuit.

The first material is a cobalt-based alloy. More preferably, the cobalt-based alloy comprises cobalt in an amount of 29 to 69 mass%. Still more preferably, the cobalt-based alloy is a cobalt-chromium alloy comprising chromium in an amount of 18 to 30 mass%.

The second material is titanium or a titanium-based alloy. More preferably, the titanium or the titanium-based alloy comprises titanium in an amount of 68 to 100 mass%.

More preferably, a ratio of an area of the second material to an area of the first material (second material/first material) = 0.2 to 10.8.

When the first material made of the cobalt-based alloy comprising cobalt in an amount of 29 to 69 mass% and the second material made of the titanium-based alloy comprising titanium in an amount of 68 to 100 mass% are used in combination, cobalt ions are eluted from the first material in an amount of 7 nmol/l to 81 $\mu$mol/l to provide a good antimicrobial property.

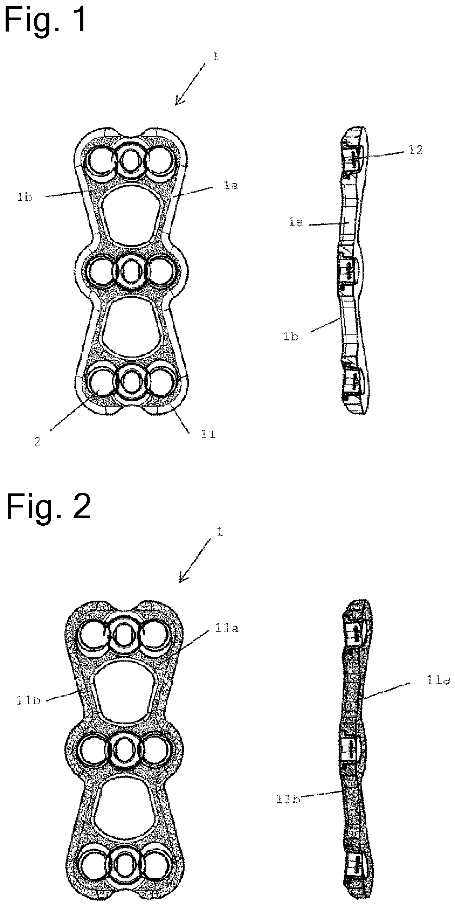

Figs. 1(a) and (b) are a plan view and a cross-sectional view, respectively, of a plate 1. The plate 1 has a plurality of screw holes 2 through which screws for fixing a plate body 11 are to be inserted. The body 11 of the plate 1 contains a second material 1a and a first material 1b that at least partially coats a surface of the second material 1a. Other than comprising the coating 1b that coats at least the surface of the body, the plate 1 can adopt components of a general plate, as required, as other components. The plate body 11 is made of the second material 1a, and at least the surface of the body 11 is coated with the first material 1b. At least the surface of the first material comprises an antimicrobial metal, at least the surface of the second material is nobler than the first material, and the first material and the second material are a combination of materials having a potential difference, such that the contact between the first material 1b and the second material 1a causes a short circuit to form an electric circuit, and antimicrobial metal ions are eluted from the first material 1b under the presence of an electrolyte to impart an antimicrobial property to the surface and/or a surrounding area of the plate body 11.

Fig. 2(a) is a plan view of a modification in which the body 11 of the plate 1 is made of a porous structure 11a composed of intertwined or agglutinated fibers comprising the second material, and a porous structure 11b composed of intertwined or agglutinated fibers comprising the first material is laminated on the porous structure 11a. Fig. 2(b) is a cross-sectional view of Fig. 2(a). Because both the first material and the second material have a fibrous shape, the surface areas of both materials increase to increase the contact area between the first material and the second material. Furthermore, because the porous structures are adopted, the amount of antibacterial metal ions eluted can be increased, and the elution of the ions can be promoted.

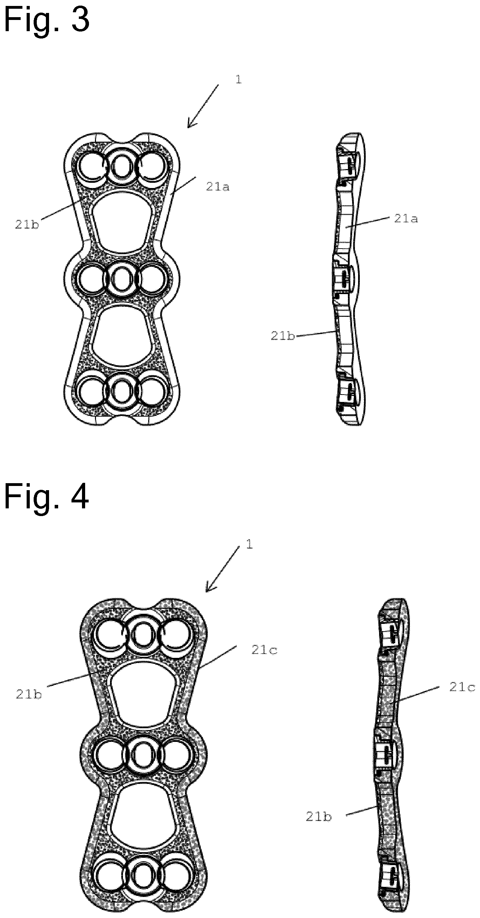

Fig. 3(a) is a plan view of a modification in which the body 11 of the plate 1 is made of a solid portion 21a formed by melting a plurality of powder particles of the second material, and a porous portion 21b formed by sintering powder particles of the second material is laminated on the solid portion 21a. Fig. 3(b) is an explanatory diagram showing the laminated state of the solid portion 21a and the porous portion 21b. While the porous portion 21b is laminated on the solid portion 21a in the illustrated embodiment, the solid portion 21a may be laminated on the porous portion 21b. To laminate the solid portion and the porous portion, compression molding, sintering, diffusion bonding, additive manufacturing using an electron beam or laser, metal injection molding, spark plasma sintering, or a combination of these methods can be suitably used. The presence of the porous portions containing the second material as powder particles can increase the surface area of the second material to increase the ratio of the surface area of the second material to the surface area of the first material, resulting in an increased amount of antibacterial metal ions eluted from the first material.

Fig. 4(a) is a plan view of a modification in which the body 11 of the plate 1 is made of a porous portion 21c formed by sintering powder particles of the second material, and a porous portion 21b formed by sintering powder particles of the first material is laminated on the porous portion 21c. Fig. 4(b) is an explanatory diagram showing the laminated state of the porous portion 21c and the porous portion 21b. To laminate the porous portion 21c and the porous portion 21b, compression molding, sintering, diffusion bonding, additive manufacturing using an electron beam or laser, metal injection molding, spark plasma sintering, or a combination of these methods can be suitably used. The presence of the porous portions of the first material and the second material can increase the surface areas of both materials to increase the contact area between the first material and the second material, resulting in an increased amount of antibacterial metal ions eluted from the first material.

EXAMPLES

The present invention will be hereinafter described in detail with examples; however, the invention is not limited to these examples.

[Example 1]

(Preparation of Samples)

A commercially available biocompatible titanium alloy (titanium content: 88 to 91 mass%) and a commercially available biocompatible cobalt-chromium alloy (cobalt content: 58 to 69 mass%, chromium content: 26 to 30 mass%) were processed into 3-mm-thick coin-shaped samples (only the titanium alloy of Sample No. 3 was processed into a flat plate-shaped sample) having the various areas shown in Table 1, and these samples were laminated in the various combinations shown in Table 1 and shorted to each other. As comparative samples, a 1-mm-thick flat plate-shaped polyethylene sample (control), a 3-mm-thick coin-shaped sample made of the cobalt-chromium alloy (Comparison 1), and a 3-mm-thick coin-shaped sample made of the titanium alloy (Comparison 2) were similarly prepared.

[Table 1]

Table 1 Sample Conditions

| Sample No. | Area Ratio* | Surface Area/mm$^2$ | |
| --- | --- | --- | --- |
| | | Titanium Alloy | Cobalt-Chromium Alloy |
| 1 | 1.0 | 518 | 518 |
| 2 | 3.3 | 1696 | 518 |
| 3 | 10.8 | 5600 | 518 |
| 4 | 0.2 | 103 | 518 |
| Comparison 1 | - | - | 518 |
| Comparison 2 | - | 518 | - |
| Control | - | Polyethylene | |
| * Area Ratio = Surface Area of Titanium Alloy/Surface Area of Cobalt-Chromium Alloy | | | |

(Antimicrobial Property Test)

An antimicrobial property was evaluated with reference to JIS Z 2801: Antibacterial products - Test for antibacterial activity and efficacy.

Each of the samples shown in Table 1 was placed in a petri dish, and 40 $\mu$l of *Staphylococcus aureus* (NBRC 12732) suspended in a nutrient broth (1/100) was added dropwise. A polyethylene film was placed over the test bacterial suspension added. The suspension was cultured at 35°C for 24 hours, and then cells were washed out from the sample. The resulting wash-out suspension was serially diluted in a 10-fold dilution series and cultured at 35°C for 24 hours using the agar plate culture method, and the number of viable cells was calculated from the number of colony forming units (CFUs) formed. The difference between the logarithmic value of the number of viable cells on the polyethylene sample as a control after 24-hour contact (Nc) and the logarithmic value of the number of viable cells on each sample (N) was determined as the antimicrobial activity value.

[Math. 1]

$$\text{Antimicrobial activity value} = \log(Nc/N)$$

Table 2 shows the number of viable cells and the antimicrobial activity value for each sample. A sample having an antimicrobial activity value of 2.0 or more was determined to have antimicrobial efficacy. The number of viable cells decreased (the antimicrobial activity value increased) as the area ratio (the surface area of the sample made of the titanium alloy/the surface area of the sample made of the cobalt-chromium alloy) increased.

[Table 2]

Table 2 Results of Antimicrobial Property Test

| Sample | Number of Viable Cells/CFU/Coupon | Antimicrobial Activity Value |
|---|---|---|
| Control: Polyethylene | $1.1 \times 10^6$ | - |
| 1 | $6.8 \times 10^3$ | 2.21 |
| 2 | $2.4 \times 10^3$ | 2.66 |
| 3 | $4.0 \times 10^2$ | 3.44 |
| Comparison 1 | $9.2 \times 10^3$ | 2.08 |

[Example 2]

(Preparation of Samples)

An implant device made of the titanium alloy and the cobalt-chromium alloy (the area ratio of the titanium alloy to the cobalt-chromium alloy was set to 0.89) was prepared (implant device 1). As a comparative example, an implant device made of only the titanium alloy having completely the same shape was similarly prepared (implant device 2).

[Table 3]

Table 3 Composition of Implant Device

| Implant Device No. | Area Ratio* | Combination of Materials | |
|---|---|---|---|
| | | Titanium Alloy | Cobalt-Chromium Alloy |
| 1 | 0.89 | ○ | ○ |
| 2 | --- | ○ | |
| * Area Ratio = Surface Area of Member Made of Titanium Alloy/Surface Area of Member Made of Cobalt-Chromium Alloy | | | |

(Antimicrobial Property Test)

Each of the implant devices was placed in a polypropylene tube, and immersed in 1 ml of *Staphylococcus aureus* (NBRC 12732) suspended in a nutrient broth (1/100). After 24 hours of culture at 35°C, the culture medium was collected. The collected culture medium was serially diluted in a 10-fold dilution series and cultured at 35°C for 24 hours using the agar plate culture method, and the number of viable cells was determined from the number of colony forming units (CFUs) formed.

Table 4 shows the number of viable cells and the antimicrobial activity value for each implant device. In the implant device 1 in which the area ratio of the titanium alloy to the cobalt-chromium alloy was appropriately set, the number of viable cells significantly decreased. In contrast, in the implant device 2 made of the titanium alloy only, a decrease in the number of viable cells was not observed.

[Table 4]

Table 4 Results of Antimicrobial Property Test for Implant Device

| Implant Device No. | Number of Viable Cells/CFU/Coupon | Antimicrobial Activity Value |
|---|---|---|
| Polyethylene | $7.6 \times 10^7$ | - |
| 1 | $1.2 \times 10^5$ | 2.80 |
| 2 | $7.0 \times 10^7$ | 0.04 |

**Claims**

1. An antimicrobial implant device made of a first material and a second material, wherein

    at least a surface of the first material comprises an antimicrobial metal; wherein the first material is a cobalt-based alloy;
    at least a surface of the second material is an antimicrobial metal, that is nobler than the first material; wherein the second material is titanium or a titanium-based alloy;
    wherein the first material and the second material have a potential difference, such that an electric circuit is formed between the first material and the second material when the device is implanted in vivo for use; and
    ions of the antimicrobial metal are eluted from the first material under a presence of an electrolyte to impart an antimicrobial property to a surface and/or a surrounding area of the first material.

2. The antimicrobial implant device according to claim 1, wherein the cobalt-based alloy comprises cobalt in an amount of 29 to 69 mass%.

3. The antimicrobial implant device according to claim 1 or 2, wherein the cobalt-based alloy is a cobalt-chromium alloy.

4. The antimicrobial implant device according to claim 3, wherein the cobalt-chromium alloy comprises chromium in an amount of 18 to 30 mass%.

5. The antimicrobial implant device according to any preceding claim, wherein the titanium or the titanium-based alloy comprises titanium in an amount of 68 to 100 mass%.

6. The antimicrobial implant device according to any one of claims 1 to 5, wherein

    the antimicrobial implant device is a plate;
    a body of the plate comprises the second material; and
    at least a part of the body of the plate is coated with the first material.

7. The antimicrobial implant device according to any one of claims 1 to 5, wherein

    the antimicrobial implant device is a plate; and
    a porous structure composed of intertwined or agglutinated fibers comprising the first material is laminated on a porous structure composed of intertwined or agglutinated fibers comprising the second material.

8. The antimicrobial implant device according to any one of claims 1 to 5, wherein

    the antimicrobial implant device is a plate; and
    a body of the plate comprises a solid portion comprising the second material and a porous portion comprising the second material wherein the solid portion and the porous portion are laminated.

9. The antimicrobial implant device according to any one of claims 1 to 5, wherein

the antimicrobial implant device is a plate;
a body of the plate comprises the second material; and
a porous portion comprising the first material is laminated on the porous portion comprising powder particles of the second material.

10. The antimicrobial implant device according to any preceding claim, wherein a ratio of a surface area of the second material to a surface area of the first material (second material/first material) is in a range of 0.2 to 10.8.

**Patentansprüche**

1. Antimikrobielle Implantatvorrichtung, die aus einem ersten Material und einem zweiten Material hergestellt ist, wobei

   wenigstens eine Oberfläche des ersten Materials ein antimikrobielles Metall umfasst; wobei das erste Material eine Kobalt-basierte Legierung ist;
   wenigstens eine Oberfläche des zweiten Materials ein antimikrobielles Metall ist, das edler als das erste Material ist; wobei das zweite Material Titan oder eine Titan-basierte Legierung ist;
   wobei das erste Material und das zweite Material eine derarte Potentialdifferenz aufweisen, dass sich ein elektrischer Stromkreis zwischen dem ersten Material und dem zweiten Material ausbildet, wenn die Vorrichtung für zur Verwendung in vivo implantiert ist; und
   Ionen des antimikrobiellen Metalls aus dem ersten Material unter einer Anwesenheit eines Elektrolyten eluiert werden, um einer Oberfläche und/oder einem Umgebungsbereich des ersten Materials eine antimikrobielle Eigenschaft zu verleihen.

2. Antimikrobielle Implantatvorrichtung nach Anspruch 1, wobei die Kobalt-basierte Legierung Kobalt in einer Menge von 29 bis 69 Massen-% umfasst.

3. Antimikrobielle Implantatvorrichtung nach Anspruch 1 oder 2, wobei die Kobalt-basierte Legierung eine Kobalt-Chrom-Legierung ist.

4. Antimikrobielle Implantatvorrichtung nach Anspruch 3, wobei die Kobalt-Chrom-Legierung Chrom in einer Menge von 18 bis 30 Massen-% umfasst.

5. Antimikrobielle Implantatvorrichtung nach einem der vorhergehenden Ansprüche, wobei das Titan oder die Titan-basierte Legierung Titan in einer Menge von 68 bis 100 Massen-% umfasst.

6. Antimikrobielle Implantatvorrichtung nach einem der Ansprüche 1 bis 5, wobei

   die antimikrobielle Implantatvorrichtung eine Platte ist;
   ein Körper der Platte das zweite Material umfasst; und
   wenigstens ein Teil des Körpers der Platte mit dem ersten Material beschichtet ist.

7. Antimikrobielle Implantatvorrichtung nach einem der Ansprüche 1 bis 5, wobei

   die antimikrobielle Implantatvorrichtung eine Platte ist; und
   eine poröse Struktur, die aus verflochtenen oder agglutinierten Fasern gebildet ist, die das erste Material umfasst, auf eine poröse Struktur laminiert ist, die aus verflochtenen oder agglutinierten Fasern gebildet ist, die das zweite Material umfasst.

8. Antimikrobielle Implantatvorrichtung nach einem der Ansprüche 1 bis 5, wobei

   die antimikrobielle Implantatvorrichtung eine Platte ist; und
   ein Körper der Platte einen festen Abschnitt, der das zweite Material umfasst, und einen porösen Abschnitt umfasst, der das zweite Material umfasst, wobei der feste Abschnitt und der poröse Abschnitt laminiert sind.

9. Antimikrobielle Implantatvorrichtung nach einem der Ansprüche 1 bis 5, wobei

   die antimikrobielle Implantatvorrichtung eine Platte ist;

ein Körper der Platte das zweite Material umfasst; und

ein poröser Abschnitt, der das erste Material umfasst, auf den porösen Abschnitt, der Pulverteilchen des zweiten Materials umfasst, laminiert ist.

10. Antimikrobielle Implantatvorrichtung nach einem der vorhergehenden Ansprüche, wobei ein Verhältnis eines Oberflächenbereichs des zweiten Materials zu einem Oberflächenbereich des ersten Materials (zweites Material/erstes Material) in einem Bereich von 0,2 bis 10,8 liegt.

**Revendications**

1. Dispositif d'implant antimicrobien constitué d'un premier matériau et d'un second matériau, dans lequel

au moins une surface du premier matériau comprend un métal antimicrobien ; le métal antimicrobien étant un alliage à base de cobalt ;

au moins une surface du second matériau est un métal antimicrobien, qui est plus noble que le premier matériau ;

le second matériau étant du titane ou un alliage à base de titane ;

le premier matériau et le second matériau ayant une différence de potentiel, de telle sorte qu'un circuit électrique est formé entre le premier matériau et le second matériau lorsque le dispositif est implanté in vivo pour utilisation ; et

des ions du métal antimicrobien étant élués à partir du premier matériau en présence d'un électrolyte pour conférer une propriété antimicrobienne à une surface et/ou à une zone environnante du premier matériau.

2. Dispositif d'implant antimicrobien selon la revendication 1, dans lequel l'alliage à base de cobalt comprend du cobalt en une quantité de 29 à 69 % en masse.

3. Dispositif d'implant antimicrobien selon la revendication 1 ou 2, dans lequel l'alliage à base de cobalt est un alliage cobalt-chrome.

4. Dispositif d'implant antimicrobien selon la revendication 3, dans lequel l'alliage cobalt-chrome comprend du chrome en une quantité de 18 à 30 % en masse.

5. Dispositif d'implant antimicrobien selon l'une quelconque des revendications précédentes, dans lequel le titane ou l'alliage à base de titane comprend du titane en une quantité de 68 à 100 % en masse.

6. Dispositif d'implant antimicrobien selon l'une quelconque des revendications 1 à 5, dans lequel

le dispositif d'implant antimicrobien est une plaque ;

un corps de plaque comprend le second matériau ; et

au moins une partie du corps de la plaque est revêtue du premier matériau.

7. Dispositif d'implant antimicrobien selon l'une quelconque des revendications 1 à 5, dans lequel

le dispositif d'implant antimicrobien est une plaque ; et

une structure poreuse composée de fibres entrelacées ou agglutinées comprenant le premier matériau est stratifiée sur une structure poreuse composée de fibres entrelacées ou agglutinées comprenant le second matériau.

8. Dispositif d'implant antimicrobien selon l'une quelconque des revendications 1 à 5, dans lequel

le dispositif d'implant antimicrobien est une plaque ; et

un corps de la plaque comprend une partie solide comprenant le second matériau et une partie poreuse comprenant le second matériau, la partie solide et la partie poreuse étant stratifiées.

9. Dispositif d'implant antimicrobien selon l'une quelconque des revendications 1 à 5, dans lequel

le dispositif d'implant antimicrobien est une plaque ;

un corps de plaque comprend le second matériau ; et

une partie poreuse comprenant le premier matériau est stratifiée sur la partie poreuse comprenant des particules de poudre du second matériau.

10. Dispositif d'implant antimicrobien selon l'une quelconque des revendications précédentes, dans lequel un rapport d'une superficie du second matériau à une superficie du premier matériau (second matériau/premier matériau) est compris entre 0,2 et 10,8.

# Fig. 1

# Fig. 2

# Fig. 3

# Fig. 4

**EP 3 424 451 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4476590 A

- JP 5175185 B